(19) Europäisches Patentamt
European Patent Office
Office européen des brevets

(11) Veröffentlichungsnummer: **0 215 860**
**B1**

(12) # EUROPÄISCHE PATENTSCHRIFT

(45) Veröffentlichungstag der Patentschrift: **13.09.89**

(51) Int. Cl.⁴: **C 07 C 177/00**, C 08 B 37/16, A 61 K 31/557

(21) Anmeldenummer: **86901814.3**

(22) Anmeldetag: **19.03.86**

(86) Internationale Anmeldenummer: **PCT/DE 86/00122**

(87) Internationale Veröffentlichungsnummer: **WO 86/05488 (25.09.86 Gazette 86/21)**

(54) **9-HALOGENPROSTAGLANDINE, VERFAHREN ZU IHRER HERSTELLUNG.**

(30) Priorität: **22.03.85 DE 3510978**

(43) Veröffentlichungstag der Anmeldung: **01.04.87 Patentblatt 87/14**

(45) Bekanntmachung des Hinweises auf die Patenterteilung: **13.09.89 Patentblatt 89/37**

(84) Benannte Vertragsstaaten: **AT BE CH DE FR GB IT LI LU NL SE**

(56) Entgegenhaltungen:
**EP-A-0 030 377**
**EP-A-0 069 696**

(73) Patentinhaber: **SCHERING AKTIENGESELLSCHAFT Berlin und Bergkamen, Müllerstrasse 170/178 Postfach 65 03 11, D-1000 Berlin 65 (DE)**

(72) Erfinder: **RADÜCHEL, Bernd, Gollanczstr. 132, D-1000 Berlin 28 (DE)**
Erfinder: **SKUBALLA, Werner, Olwenstr. 13, D-1000 Berlin 28 (DE)**
Erfinder: **VORBRÜGGEN, Helmut, Wilkestr. 7, D-1000 Berlin 27 (DE)**
Erfinder: **LOGE, Olaf, Bekassinenweg 37, D-1000 Berlin 27 (DE)**
Erfinder: **SCHILLINGER, Ekkehard, Im Amseltal 50, D-1000 Berlin 28 (DE)**

EP 0 215 860 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

## Beschreibung

Die vorliegende Erfindung betrifft (5Z,13E)-(9R,11R,15S)-9-Halogen-15-cycloalkyl-11,15-dihydroxy-16,17,18,19,20-pentanor-5,13-prostadiensäure-Derivate, deren physiologisch verträgliche Salze und deren Clathrate, Verfahren zu ihrer Herstellung und diese enthaltende pharmazeutische Zusammensetzungen.

In den deutschen Offenlegungsschriften 2 950 027 und 3 126 924 werden 9-Halogen-prostanderivate der nachstehenden Formel

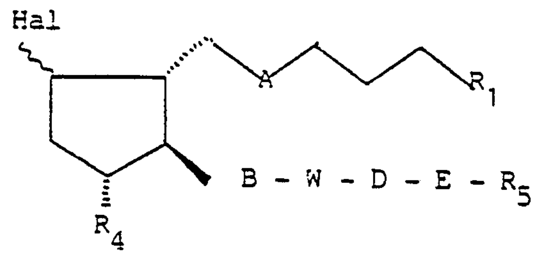

beansprucht, in der

Hal Fluor oder Chlor,

$R_1$ den Rest $CH_2OH$ oder

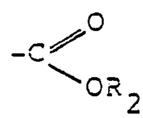

mit $R_2$ in der Bedeutung eines Wasserstoffatoms, eines Alkyl-, Cycloalkyl-, Aryl- oder heterocyclischen Restes oder $R_1$ den Rest

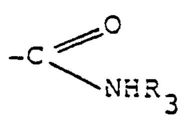

mit $R_3$ in der Bedeutung eines Säurerestes oder des Restes $R_2$ und

A eine $-CH_2-CH_2-$ oder cis-$CH=CH$-Gruppe,

B eine $-CH_2-CH_2-$, oder trans-$CH=CH$- oder eine $-C\equiv C$-Gruppe,

W eine freie oder funktionell abgewandelte Hydroxymethylengruppe wobei die OH-Gruppe α- oder β-ständig sein kann,

D und E gemeinsam eine direkte Bindung oder

D eine gerad- oder verzweigtkettige Alkylengruppe mit 1 - 10 C-Atomen, die gegebenenfalls durch Fluoratome substituiert sein kann,

E ein Sauerstoff- oder Schwefelatom eine direkte Bindung, eine $-C\equiv C$-Bindung oder eine $-CR_6=CR_7$-Gruppe darstellt, wobei $R_6$ und $R_7$ sich unterscheiden und ein Wasserstoffatom, ein Chloratom oder eine Alkylgruppe bedeuten,

$R_4$ eine freie oder funktionell abgewandelte Hydroxygruppe,

$R_5$ ein Wasserstoffatom, eine Alkyl-, eine Halogen-substituierte Alkyl-, eine Cycloalkyl-, eine gegebenenfalls substituierte Aryl- oder heterocyclische Gruppe und falls $R_2$ die Bedeutung eines Wasserstoffatoms hat, deren Salze mit physiologisch verträglichen Basen bedeuten.

Die Prostaglandinderivate aus DE-OS-2 950 027 und 3 126 924 sind geeignet, nach einmaliger enteraler oder parenteraler Applikation eine Menstruation zu induzieren oder eine Schwangerschaft zu unterbrechen. Sie eignen sich sowohl zur Synchronisation des Sexualzyklus bei weiblichen Säugetieren wie Kaninchen, Rindern, Pferden oder Schweinen als auch zur Cervixdilatation als Vorbereitung für diagnostische oder therapeutische Eingriffe.

Sie hemmen die Magensäuresekretion, zeigen einen zytoprotektiven und ulcusheilenden Effekt und wirken damit den unerwünschten Folgen nichtsteroidaler Entzündungshemmstoffe (Prostaglandinsynthese - Inhibitoren) entgegen.

Einige dieser Verbindungen wirken auch blutdrucksenkend regulierend bei Herzrhythmusstörungen und hemmend auf die Plättchenaggregation.

Die 9-Halogen-15-cycloalkylprostaglandinderivate wurden weder in der DE-OS-2 950 027 noch in der DE-OS-3 126 924 namentlich beschrieben und zeigen ein gegenüber den Verbindungen aus den genannten

Offenlegungsschriften abweichendes pharmakologisches Verhalten auf das später noch eingegangen wird.

Die Erfindung betrifft somit 9-Halogen-15-cycloalkylprostaglandinderivate der Formel I

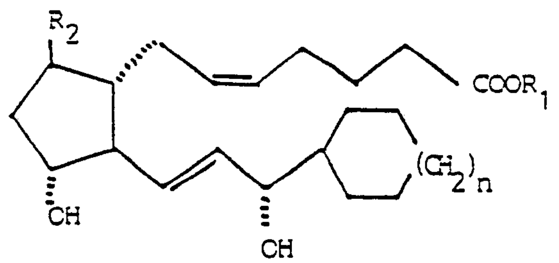

(I),

worin

$R_1$ Wasserstoff oder Methyl,
$R_2$ Fluor oder Chlor,
n 0 oder 1 bedeuten und, falls $R_1$ Wasserstoff bedeutet, deren Salze mit physiologisch verträglichen Basen oder deren Cyclodextrinclathrate.

Als Alkylgruppen $R_3$ kommen gerad- und verzweigtkettige Alkylreste mit 1 - 6 C-Atomen in Betracht wie beispielsweise Methyl, Ethyl, Propyl, Butyl, Isopropyl, Isobutyl, Pentyl, Isopentyl, Hexyl. Bevorzugte Reste sind Methyl, Ethyl, Propyl und Isopropyl.

Zur Salzbildung sind anorganische und organische Basen geeignet, wie sie dem Fachmann zur Bildung physiologisch verträglicher Salze bekannt sind. Beispielsweise genannt seien Alkalihydroxide, wie Natrium- und Kaliumhydroxid, Erdalkalihydroxide wie Calciumhydroxid, Ammoniak, Amine, wie Äthanolamin, Diäthanolamin, Triäthanolamin, N-Methylglucamin, Morpholin, Tris-(hydroxymethyl)-methylamin usw.

Die Erfindung betrifft ferner ein Verfahren zur Herstellung der 9-Halogen-15-cycloalkylprostaglandinderivate der Formel I, dadurch gekennzeichnet, daß man eine Verbindung der Formel II,

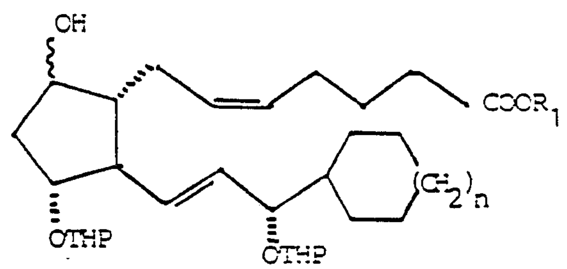

(II),

worin die OH-Gruppe α- oder β-ständig sein kann, $R_1$ Methyl bedeutet, n die oben angegebene Bedeutung hat und THP Tetrahydropyranyl bedeutet, über einen intermediären 9-Sulfonsäureester mit einem Halogenid der Formel III $R_3Hal$, worin $R_3$ Lithium, Natrium oder Kalium bedeutet, wenn Hal Chlor darstellt und $R_3$ Tetra-($C_1$-$C_6$-alkyl)-ammonium bedeutet, wenn Hal Fluor darstellt, umsetzt und gegebenenfalls anschließend in den erhaltenen Reaktionsprodukten in beliebiger Reihenfolge geschützte Hydroxygruppen freisetzt, die veresterte Carboxylgruppe ($R_1=CH_3$) verseift und/oder die freie Carboxylgruppe ($R_1=H$) in ein Salz oder Clathrat überführt.

Die Umsetzung der Verbindungen der allgemeinen Formel II zu einem 9-Sulfonsäureester erfolgt in an sich bekannter Weise mit einem Alkylsulfonylchlorid oder Arylsulfonylchlorid in Gegenwart eines Amins wie beispielsweise Pyridin oder Triäthylamin bei Temperaturen zwischen -60°C und +100°C, vorzugsweise -20°C bis +50°C. Die nucleophile Substitution des 9-Sulfonats durch ein Chloratom erfolgt mit einem Alkalichlorid, vorzugsweise Lithiumchlorid und durch ein Fluoratom mit Tetra-($C_1$-C-alkyl)-ammoniumfluorid, vorzugsweise Tetrabutylammoniumfluorid, in einem inerten Lösungsmittel wie beispielsweise Dimethylformamid, Dimethylacetamid, Dimethylsulfoxid, Dimethoxyäthan, Tetrahydrofuran usw. bei Temperaturen zwischen 0°C und 100°C, vorzugsweise 20°C bis 80°C.

Die Abspaltung des Tetrahydropyranylrestes wird in einer wäßrigen Lösung einer organischen Säure wie z. B. Oxalsäure, Essigsäure, Propionsäure u.a., oder in einer wäßrigen Lösung einer anorganischen Säure, wie z. B. Salzsäure, durchgeführt. Zur Verbesserung der Löslichkeit wird zweckmäßigerweise ein mit Wasser mischbares inertes organisches Lösungsmittel zugesetzt. Geeignete organische Lösungsmittel sind z. B. Alkohole, wie Methanol und Ethanol und Äther wie Dimethoxyäthan, Dioxan und Tetrahydrofuran. Tetrahydrofuran wird bevorzugt angewendet. Die Abspaltung wird vorzugsweise bei Temperaturen zwischen 20°C und 80°C durchgeführt.

Die Prostaglandinderivate der Formel I mit $R_1$ in der Bedeutung eines Wasserstoffatoms können mit

geeigneten Mengen der entsprechenden anorganischen Basen unter Neutralisierung in ein Salz überführt werden. Beispielsweise erhält man bei Lösen der entsprechenden PG-Säuren in Wasser, welches die stöchiometrische Menge der Base enthält, nach Abdampfen des Wassers oder nach Zugabe eines mit Wasser mischbaren Lösungsmittels, z. B. Alkohol oder Aceton, das feste anorganische Salz.

Zur Herstellung eines Aminsalzes, die in üblicher Weise erfolgt, wird die PG-Säure z. B. in einem geeigneten Lösungsmittel, beispielsweise Äthanol, Aceton, Diäthyläther, Acetonitril oder Benzol gelöst und mindestens die stöchiometische Menge des Amins dieser Lösung zugesetzt. Dabei fällt das Salz gewöhnlich in fester Form an oder wird nach Verdampfen des Lösungsmittels in üblicher Weise isoliert.

Zur Herstellung der Clathrate werden die Verbindungen der Formel I in einem pharmakologisch unbedenklichen Alkohol, vorzugsweise Ethanol, aufgelöst und zu wäßrigen Lösungen von α-, β- oder γ-Cyclodextrin, vorzugsweise β-Cyclodextrin, bei 60°C zugesetzt. Nach dem Abkühlen kristallisieren die entsprechenden Clathrate aus und können durch Absaugen und Trocknen als feste, frei fließende Kristalle isoliert werden.

Die Ausgangsverbindungen der Formel II werden in der Deutschen Offenlegungsschrift DE-OS-2 515 770 beschrieben.

Die erfindungsgemäßen 15-Cycloalkylprostaglandine der Formel I zeichnen sich durch $PGD_2$-typische Eigenschaften aus, d.h. sie binden gut am $PGD_2$-Rezeptor, dagegen schlecht am $PGE_2$- und $PGI_2$-Rezeptor. Untersuchungen an der Rindercoronararterie und an der Kaninchenpulmonalarterie weisen ebenfalls auf ein $PGD_2$-Wirkprofil der Verbindungen der Formel I hin.

Die Verbindungen wirken nach i.v.-Infusion an der Ratte blutdrucksenkend.

Die erfindungsgemäßen Wirkstoffe hemmen die Magensäuresekretion, zeigen einen zytoprotektiven und ulcusheilenden Effekt und wirken damit den unerwünschten Folgen nichtsteroidaler Entzündungshemmstoffe (Prostaglandinsynthese - Inhibitoren) entgegen. Sie wirken regulierend bei Herzrhythmusstörungen und hemmend auf die Plättchenaggregation.

Für die medizinische Anwendung können die Wirkstoffe in eine für die Inhalation, für orale paranterale oder lokale (z. B. vaginale) Applikation geeignete Form überführt werden.

Zur Inhalation werden zweckmäßigerweise Ärosollösungen hergestellt.

Für die orale Applikation sind beispielsweise Tabletten, Dragees oder Kapseln geeignet.

Für die parenterale Verabreichung werden sterile, injizierbare wäßrige oder ölige Lösungen benutzt.

Für die vaginale Applikation sind z. B. Zäpfchen geeignet und üblich.

Die Erfindung betrifft damit auch Arzneimittel aus Basis der Verbindungen der allgemeinen Formel I und der üblichen Hilfs- und Trägerstoffe.

Die erfindungsgemäßen Wirkstoffe sollen in Verbindung mit den in der Galenik bekannten und üblichen Hilfsstoffen, z. B. zur Herstellung von pharmazeutischen Präparaten dienen. Die Präparate können 0,01 - 50 mg der aktiven Verbindung enthalten.

Die folgenden Beispiele sollen die Erfindung näher erläutern, ohne daß damit eine Begrenzung vorgenommen wird.

**Beispiel 1**

(5Z,13E)-(9R,11R,15S)-9-Chlor-15-cyclohexyl-11,15-dihydroxy-16,17,18,19,20-pentanor-5,13-prostadiensäuremethylester

Zu einer Lösung von 1,098 g (5Z,13E)-(9S,11R, 15S)-15-Cyclohexyl-9-hydroxy-11,15-bis(tetrahydropyran-2-yloxy)-16,17,18,19,20-pentanor-5,13-prostadiensäuremethylester in 10 ml Pyridin gibt man bei 0°C 191 mg p-Toluolsulfonsäurechlorid und nach 24 Stunden weitere 191 mg. Man läßt insgesamt 48 Stunden bei 0°C stehen, versetzt dann mit 0,1 ml Wasser rührt 2 Stunden, verdünnt mit 200 ml Äther und wäscht nacheinander mit verdünnter Schwefelsäure, Wasser und Sole, trocknet über Magnesiumsulfat, dampft ein und erhält 1,20 g des 9α-Tosylats als farbloses Öl.

IR ($CHCl_3$): 2945, 2872, 1735, 1366, 1240, 978/cm.

Eine Lösung von 1,05 g des 9α-Tosylats in 45 ml Dimethylformamid wird mit 600 mg Lithiumchlorid 4 Stunden bei 65°C gerührt. Man verdünnt mit Sole, extrahiert mit Äther/Hexan (1 : 1), wäscht den Extrakt mit Wasser und Sole, trocknet über Magnesiumsulfat und dampft im Vakuum ein. Den Rückstand reinigt man durch Chromatographie an Kieselgel mit Toluol-Ethylacetat (95 : 5 bis 80 : 20). Man erhält 520 mg der 9β-Chlorverbindung, die man zur Abspaltung der Tetrahydropyranylätherschutzgruppen mit 20 ml einer Mischung aus Essigsäure-Wasser-Tetrahydrofuran (65/35/10) 18 Stunden bei 32°C rührt. Nach Eindampfen der Lösung im Vakuum chromatographiert man den Rückstand an Kieselgel. Mit Dichlormethan-Aceton (9 : 1) als Elutionsmittel erhält man 285 mg der Titelverbindung als farbloses Öl.

IR ($CHCl_3$): 3600, 3410, 3000, 2932, 2859, 1732, 972/cm.

**Beispiel 2**

(5Z,13E)-(9R,11R,15S)-9-Chlor-15-cyclohexyl-11,15-dihydroxy-16,17,18,19,20-pentanor-5,13-prostadiensäure

220 mg des Methylesters, der nach Beispiel 1 erhalten wurde, wird mit einer Lösung aus 100 mg Kaliumhydroxid, 0,5 ml Wasser, und 5 ml Ethanol 5 Stunden bei 20°C gerührt. Man engt im Vakuum ein, verdünnt mit 20 ml Wasser stellt mit Zitronensäure auf pH 4 und extrahiert mit Dichlormethan. Die Extrakte werden mit Wasser gewaschen, über Magnesiumsulfat getrocknet und im Vakuum eingedampft. Den Rückstand reinigt man durch Chromatographie an Kieselgel, wobei man mit Dichlormethan-Methanol (95 : 5) eluiert. Man erhält 192 mg der Titelverbindung als farbloses Öl.

IR ($CHCl_3$): 3600, 3450, 2955, 2859, 1710, 974/cm.

**Beispiel 3**

(5Z,13E)-(9R,11R,15S)-9-Chlor-15-cyclopentyl-11,15-dihydroxy-16,17,18,19,20-pentanor-5,13-prostadiensäuremethylester

Man geht aus von 1,05 g (5Z,13E)-(9S,11R,15S)-15-Cyclopentyl-9-hydroxy-11,15-bis(tetrahydroparan-2-yloxy)-16,17,18 19,20-pentanor-5,13-prostadiensäuremethylester und verfährt wie in Beispiel 1 beschrieben. Man erhält 242 mg der Titelverbindung als farbloses Öl.

IR ($CHCl_3$): 3600, 3405, 2998, 2935, 2858, 1735, 978/cm.

**Beispiel 4**

(5Z,13E)-(9R,11R 15S)-9-Chlor-15-cyclopentyl-11,15-dihydroxy-16,17,18,19,20-pentanor-5,13-prostadiensäure

180 mg des nach Beispiel 3 erhaltenen Methylesters verseift man nach der in Beispiel 2 angegebenen Methode und erhält 135 mg der Titelverbindung als Öl.

IR ($CHCl_3$): 3600, 3450, 2927, 2860, 1709, 976/cm.

**Beispiel 5**

(5Z,13E)-(9R,11R,15S)-15-Cyclohexyl-11,15-dihydroxy-9-fluor-16,17,18,19,20-pentanor-5,13-prostadiensäuremethylester

Zu einer Lösung von 4,2 g (5Z,13E)-(9S,11R,15S)-15-Cyclohexyl-9-tosyloxy-11,15-bis(tetrahydropyran-2-yloxy)-16,17,18,19,20-pentanor-5,13-prostadiensäuremethylester (hergestellt nach Beispiel 1) in 70 ml Dimethylsulfoxid fügt man 12 g Tetrabutylammoniumfluorid (getrocknet durch mehrmaliges Abdestillieren von Toluol im Vakuum bei 50°C) und rührt 2 Stunden bei 22°C unter Argon. Anschließend verdünnt man mit 400 ml Wasser, extrahiert dreimal mit je 150 ml Äther/Hexan (2 : 1), wäscht die Extrakte mit Sole, trocknet über Magnesiumsulfat und dampft im Vakuum ein. Den Rückstand reinigt man an Kieselgel und eluiert mit Hexan-Ethylacetat (10 : 1) 1,03 g der 9β-Fluorverbindung, die zur Abspaltung der Schutzgruppen 18 Stunden bei 22°C mit 25 ml einer Mischung aus Essigsäure-Wasser-Tetrahydrofuran (65/35/10) gerührt werden. Nach Eindampfen der Lösung und chromatographischer Reinigung an Kieselgel mit Dichlormethan-Aceton (10 : 1) erhält man 830 mg der Titelverbindung als farbloses Öl.

IR ($CHCl_3$): 3600, 3410, 2998, 2932, 2865, 1729, 976/cm.

**Beispiel 6**

(5Z,13E)-(9R,11R,15S)-15-Cyclohexyl-11,15-dihydroxy-9-fluor-16,17,18 19,20-pentanor-5,13-prostadiensäure

Man verfährt gemäß Beispiel 2 und erhält aus 500 mg des Methylesters, der nach Beispiel 5 hergestellt wurde, 425 mg der Titelverbindung als farbloses Öl.

IR ($CHCl_3$): 3600, 3405, 2930, 2858, 1710, 976/cm.

**Beispiel 7**

(5Z,13E)-(9R,11R, 15S)-15-Cyclopentyl-11,15-dihydroxy-9-fluor-16,17,18,19,20-pentanor-5,13-prostadiensäuremethylester

Zu einer Lösung von 1,07 g (5Z,13E)-(9S,11R,15S)-15-Cyclopentyl-9-hydroxy-11,15-bis(tetrahydropyran-2-yloxy)-16,17,18,19,20-pentanor-5,13-prostadiensäuremethylester in 10 ml Pyridin gibt man bei 0°C 191 mg p-Toluolsulfonsäurechlorid, rührt bei 0°C weiter und versetzt nach 24 Stunden mit weiteren 191 mg Säurechlorid. Nach 48 Stunden gibt man 0,1 ml Wasser, rührt 2 Stunden, verdünnt mit Äther und wäscht nacheinander mit verdünnter Schwefelsäure, Wasser und Sole, trocknet über Magnesiumsulfat, dampft ein und erhält 1,12 g des 9α-Tosylats als farbloses Öl.

IR ($CHCl_3$): 2952, 2878, 1733, 1359, 1241, 976/cm.

Eine Lösung von 1 g des 9 α-Tosylats in 20 ml Dimethylsulfoxid versetzt man mit 3 g Tetrabutylammoniumfluorid, rührt 2 Stunden bei Raumtemperatur, verdünnt dann mit Wasser und extrahiert dann mehrere Male mit Äther/Hexan (2 : 1). Die vereinigten Extrakte werden mit Sole gewaschen, über Magnesiumsulfat getrocknet und im Vakuum eingedampft. Den Rückstand reinigt man an Kieselgel und eluiert mit Hexan-Ethylacetat (85 : 15) 205 mg der 9β-Fluorverbindung, die zur Abspaltung der Schutzgruppen 18 Stunden bei Raumtemperatur mit 5 ml einer Mischung aus Essigsäure-Wasser-Tetrahydrofuran (65/35/10) gerührt werden. Nach Eindampfen der Lösung und chromatographischer Reinigung an Kieselgel mit Dichlormethan-Aceton (85 : 15) erhält man 110 mg der Titelverbindung als farbloses Öl.

IR ($CHCl_3$): 3600, 3408, 2998, 2929, 2854, 1733, 976/cm.

**Beispiel 8**

(5Z,13E)-(9R,11R,15S)-15-Cyclopentyl-11,15-dihydroxy-9-fluor-16,17,18 19,20-pentanor-5,13-prostadiensäure

Zu einer Lösung von 85 mg des nach Beispiel 7 hergestellten Methylesters in 5 ml Ethanol gibt man 50 mg Kaliumhydroxid gelöst in 0,5 ml Wasser und rührt 5 Stunden bei 20°C. Nach Einengen im Vakuum versetzt man mit 10 ml Wasser, säuert mit Zitronensäure auf pH 3 an und extrahiert mehrmals mit Dichlormethan, trocknet über Magnesiumsulfat und dampft im Vakuum ein. Man erhält 72 mg der Titelverbindung als farbloses Öl.

IR ($CHCl_3$): 3600, 3430, 2931, 2859, 1710, 974/cm.

**Beispiel 9**

Tris(hydroxymethyl)-aminomethansalz von (5Z,13E)-(9R,11R,15S)-9-Chlor-15-cyclohexyl-11,15-dihydroxy-16,17,18,19,20-pentanor-5,13-prostadiensäure

Zu einer Lösung von 250 mg (5Z,13E)-(9R,11R,15S)-9-Chlor-15-cyclohexyl-11,15-dihydroxy-16,17,18,19,20-pentanor-5,13-prostadiensäure in 40 ml Acetonitril gibt man bei 65°C eine Lösung von 75 mg Tris(hydroxymethyl)-aminomethan in 0,25 ml Wasser. Unter Rühren läßt man auf 20°C abkühlen und saugt das Lösungsmittel ab. Man erhält 205 mg der kristallinen Titelverbindung, Fp. 118 - 120°C.

IR (KBr): 2965, 1655, 1420, 976/cm.

**Patentansprüche**

1. 9-Halogen-15-cycloalkylprostaglandinderivate der Formel I

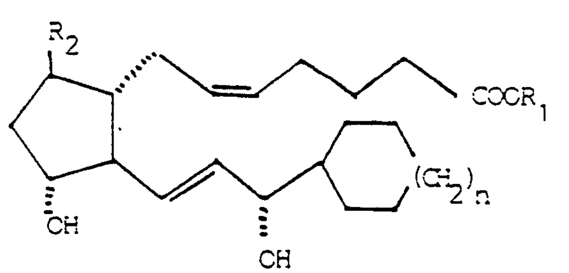

(I),

worin

$R_1$ Wasserstoff oder Methyl
$R_2$ Fluor oder Chlor,
n 0 oder 1 bedeuten und, falls $R_1$ Wasserstoff bedeutet, deren Salze mit physiologisch verträglichen Basen oder deren Cyclodextrinclathrate.

2. Verfahren zur Herstellung der 9-Halogen-15-cyclolalkyl-prostaglandinderivate der Formel I, dadurch gekennzeichnet, daß man eine Verbindung der Formel II,

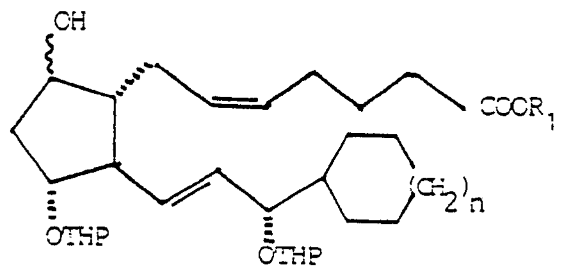

(II),

worin die OH-Gruppe α- oder β-ständig sein kann, $R_1$ Methyl bedeutet, n die oben angegebene Bedeutung hat und THP Tetrahydropyranyl bedeutet, über einen intermediären 9-Sulfonsäureester mit einem Halogenid der Formel III $R_3$ Hal, worin $R_3$ Lithium, Natrium oder Kalium bedeutet, wenn Hal Chlor darstellt und $R_3$ Tetra-($C_1$-$C_6$-alkyl)-ammonium bedeutet, wenn Hal Fluor darstellt, umsetzt und gegebenenfalls anschließend in den erhaltenen Reaktionsprodukten in beliebiger Reihenfolge geschützte Hydroxygruppen freisetzt, die veresterte Carboxylgruppe ($R_1 = CH_3$) verseift und/oder die freie Carboxylgruppe ($R_1 = H$) in ein Salz oder Clathrat überführt.

3. Arzneimittel bestehend aus einer oder mehreren Verbindungen des Anspruchs 1 und üblichen Hilfs- und Trägerstoffen.

4. (5Z,13E)-(9R,11R,15S)-9-Chlor-15-cyclohexyl-11,15-dihydroxy-16,17,18,19,20-pentanor-5,13-prostadiensäure.

5. (5Z,13E)-(9R,11R,15S)-9-Chlor-15-cyclopentyl-11,15-dihydroxy-16,17,18,19,20-pentanor-5,13-prostadiensäure.

6. (5Z,13E)-(9R,11R,15S)-15-Cyclohexyl-11,15-dihydroxy-9-fluor-16,17,18,19,20-pentanor-5,13-prostadiensäure.

7. (5Z,13E)-(9R,11R,15S)-15-Cyclopentyl-11,15-dihydroxy-9-fluor-16,17,18,19,20-pentanor-5,13-prostadiensäure.

8. Tris(hydroxymethyl)-aminomethansalz von (5Z,13E)-(9R,11R,15S)-9-Chlor-15-cyclohexyl-11,15-dihydroxy-16,17,18,19,20-pentanor-5,13-prostadiensäure.

## Claims

1. 9-Halo-15-cycloalkylprostaglandin derivatives of formula I

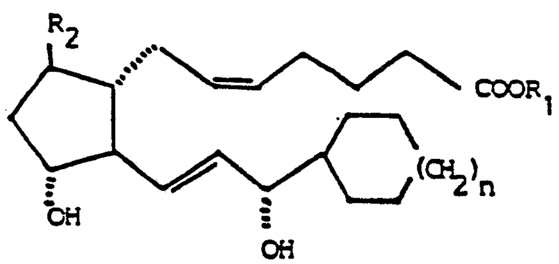

(I)

wherein

$R_1$ is hydrogen or methyl,
$R_2$ is fluorine or chlorine, and
n is 0 or 1, and when $R_1$ is hydrogen, their salts with physiologically acceptable bases or their cyclodextrin clathrates.

2. Process for the preparation of 9-halo-15-cycloalkylprostaglandin derivatives of formula I, characterised in that a compound of formula II

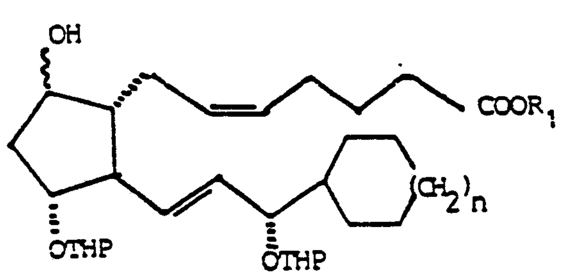

(II)

in which the OH group is in the α- or β-configuration, $R_1$ is methyl, n has the meaning given above and THP is tetrahydropyranyl, is treated, via an intermediate 9-sulphonate ester, with a halide of formula III $R_3Hal$, wherein $R_3$ is lithium, sodium or potassium when Hal is chlorine, or tetra($C_1$-$C_4$-alkyl)ammonium when Hal is fluorine, and optionally, subsequently in the resulting reaction product, in any preferred order, protected hydroxy groups are freed, the esterified carboxy group ($R_1$ = $CH_3$) is saponified, and/or the free carboxy group ($R_1$ = H) is converted to a salt or clathrate.

3. Pharmaceuticals containing one or more compounds according to claim 1 and conventional additives and carriers.

4. (5Z,13E)-(9R,11R,15S)-g-Chloro-15-cyclohexyl-11,15-dihydroxy-16,17,18,19,20-pentanor-5,13-prostadienoic acid.

5. (5Z,13E)-(9R,11R,15S)-9-Chloro-15-cyclopentyl-11,15-dihydroxy-16,17,18,19,20-pentanor-5,13-prostadienoic acid.

6. (5Z,13E)-(9R,11R,15S)-15-Cyclohexyl-11,15-dihydroxy-9-fluoro-16,17,18,19,20-pentanor-5,13-prostadienoic acid.

7. (5Z,13E)-(9R,11R, 15S)-15-Cyclopentyl-11,15-dihydroxy-9-fluoro-16,17,18,19,20-pentanor-5,13-prostadienoic acid.

8. Tris(hydroxymethyl)aminomethyl salt of (5Z,13E)-(9R,11R,15 )-9-chloro-15-cyclohexyl-11,15-dihydroxy-16,17,18,19,20-pentanor-5,13-prostadienoic acid.

**Revendications**

1. 9-Halogéno-15-cycloalkylprostaglandines de formule I ci-dessous:

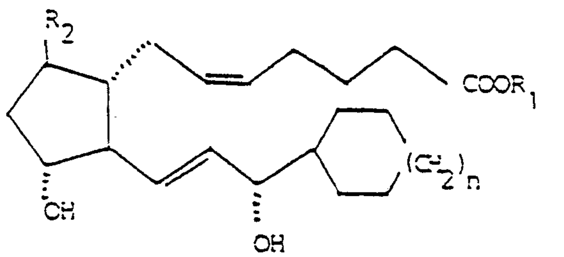

(I),

dans laquelle:

$R_1$ désigne l'hydrogène ou le groupe méthyle,
$R_2$ désigne le fluor ou le chlore et
n est le nombre 0 ou 1, ainsi que, si $R_1$ est l'hydrogène, leurs sels formés avec des bases physiologiquement compatibles ou leurs clathrates de cyclodextrine.

2. Procédé de préparation des 9-halogéno-15-cycloalkylprostaglandines de formule I selon la revendication 1, procédé caractérisé en ce que l'on fait réagir un composé de formule II:

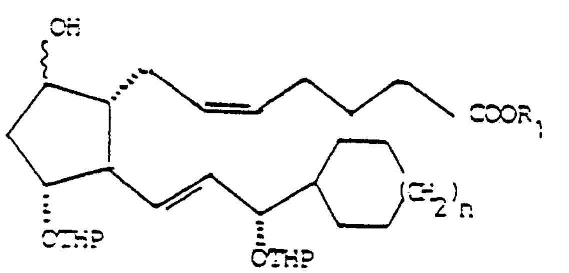

(II),

(dont le groupe OH peut être à la position α ou β , $R_1$ est le groupe méthyle, n le nombre 0 ou 1 et THP désigne le groupe tétrahydropyranyle), en passant par l'intermédiaire d'un ester d'acide sulfonique à la position 9, avec un halogénure de formule III $R_3Hal$, $R_3$ étant le lithium, le sodium ou le potassium si Hal est le chlore ou un groupe tétraalkylammonium à alkyles en $C_1$ à $C_6$ si Hal est le chlore, puis le cas échéant, et en opérant dans un ordre quelconque, on libère les groupes hydroxyliques protégés des produits de réaction obtenus, on saponifie le groupe carboxylique estérifié ($R_1$ étant alors le groupe $CH_3$) et/ou on transforme le groupe carboxylique libre ($R_1$ étant l'hydrogène) en un sel ou clathrate.

3. Médicament comprenant un ou plusieurs composés selon la revendication 1 avec des adjuvants et véhicules usuels.

4. (5Z,13E)-(9R,11R, 15S)-9-Chloro-15-cyclohexyl-11,15-dihydroxy-16,17,18,19,20-pentanor-5,13-prostadiénoïque.

5. (5Z,13E)-(9R,11R,15S)-9-Chloro-15-cyclopentyl-11,15-dihydroxy-16,17,18,19,20-pentanor-5,13-prostadiénoïque.

6. (5Z,13E)-(9R,11R,15S)-15-Cyclohexyl-11,-15-dihydroxy-9-fluoro-16,17,18,19,20-pentanor-5,13-prostadiénoïque.

7. (5Z,13E)-(9R,11R, 15S)-15-Cyclopentyl-11,-15-dihydroxy-9-fluoro-16,17,18,19,20-pentanor-5,13-prostadiénoïque.

8. (5Z,13E)-(9R,11R, 15S)-9-Chloro-15-cyclohexyl-11,15-dihydroxy-16,17,18,19,20-pentanor-5,13-prostadiénoate de tris(hydroxyméthyl)-aminométhane.